(19) Europäisches Patentamt
European Patent Office
Office européen des brevets

(11) Publication number: **0 103 126**
**B1**

(12) # EUROPEAN PATENT SPECIFICATION

(45) Date of publication of patent specification: **04.03.87**

(51) Int. Cl.[4]: **C 07 D 239/42,** C 07 C 121/38, C 07 C 120/00

(21) Application number: **83107398.6**

(22) Date of filing: **27.07.83**

(54) **Process for preparing 4-amino-5-dialkoxymethylpyrimidine derivatives.**

(30) Priority: **10.09.82 JP 156687/82**

(43) Date of publication of application: **21.03.84 Bulletin 84/12**

(45) Publication of the grant of the patent: **04.03.87 Bulletin 87/10**

(84) Designated Contracting States: **AT BE FR IT LU NL SE**

(56) References cited:
**DE-B-1 016 266**
**DE-C- 937 057**
**US-A-4 319 024**

**CHEMISCHE BERICHTE, 106, no. 11, 1973, Gesellschaft Deutscher Chemiker, Weinheim/Bergstrasse H. BREDERECK et al. "Synthese des Pyrimido (4,5-d)pyrimidins" pages 3743-3752**

**COMPREHENSIVE ORGANIC CHEMISTRY, Vol. 4, page 89**

(73) Proprietor: **Ube Industries, Ltd.**
**12-32, Nishihonmachi 1-chome**
**Ube-shi, Yamaguchi-ken (JP)**

(72) Inventor: **Fujii, Kozo c/o Head Office**
**Ube Industries Ltd. 12-32, Nishi-honmachi 1-chome**
**Ube-shi Yamaguchi-ken (JP)**
Inventor: **Nishihira, Keigo c/o Head Office**
**Ube Industries Ltd. 12-32, Nishi-honmachi 1-chome**
**Ube-shi Yamaguchi-ken (JP)**
Inventor: **Sawada, Hiroyuki c/o Head Office**
**Ube Industries Ltd. 12-32,Nishi-honmachi 1-chome**
**Ube-shi Yamaguchi-ken (JP)**
Inventor: **Tanaka, Shuji c/o Head Office**
**Ube Industries Ltd. 12-32, Nishi-honmachi 1-chome**
**Ube-shi Yamaguchi-ken (JP)**
Inventor: **Nakai, Mamoru Central Research Laboratories**
**Ube Industries Ltd. 1978-5, Oaza Kogushi**
**Ube-shi Yamagushi-ken (JP)**
Inventor: **Yoshida, Hiroshi Central Research Laboratories**
**Ube Industries Ltd. 1978-5, Oaza Kogushi**
**Ube-shi Yamagushi-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Inoue, Teruhiko Central Research Laboratories**
**Ube Industries, Ltd. 1978-5, Oaza Kogushi**
**Ube-shi Yamagushi-ken (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

**Description**

This invention relates to a novel process for preparing a 4-amino-5-dialkoxymethylpyrimidine derivative.

The 4-amino-5-dialkoxymethylpyrimidine derivative is utilized as an intermediate for the synthesis of Vitamin-$B_1$ and its analogues.

As a method for the preparation of a 4-amino-5-dialkoxymethylpyrimidine derivative, the following method has been disclosed in Chem. Ber. *106*, 3743 (1973):

First, a 4,6-dichloro-5-formylpyrimidine is reacted with ammonia. Then, the so obtained 4-amino-5-formyl-6-chloropyrimidine is reacted with hydrogen to form a 4-amino-5-formylpyrimidine, which is subsequently reacted with a trialkoxymethane to prepare the desired 4-amino-5-dialkoxymethylpyrimidine. This method has industrial problems in that many and complicated reaction steps are involved and that the synthesis of the starting pyrimidine derivative is not easy.

US—A—4 319 024 discloses the reaction of a β-alkoxyacrilonitrile with urea to form cytosine derivatives. The β-alkoxyacrilonitriles are obtained by an alkyl etherification reaction in which an alkyl halide is used. This reaction cannot avoid the formation of byproducts. Furthermore, in COMPREHENSIVE ORGANIC CHEMISTRY, Vol. 4, page 89, it is emphasized that the condensation of a three-carbon unit with an N—C—N fragment appears to be the most widely used method for forming the pyrimidine nucleus.

The present inventors have made earnest studies to establish a process by which the 4-amino-5-dialkoxymethylpyrimidine derivative can advantageously be prepared industrially.

The reaction according to DE—B—1 016 266 may be summarized as follows:

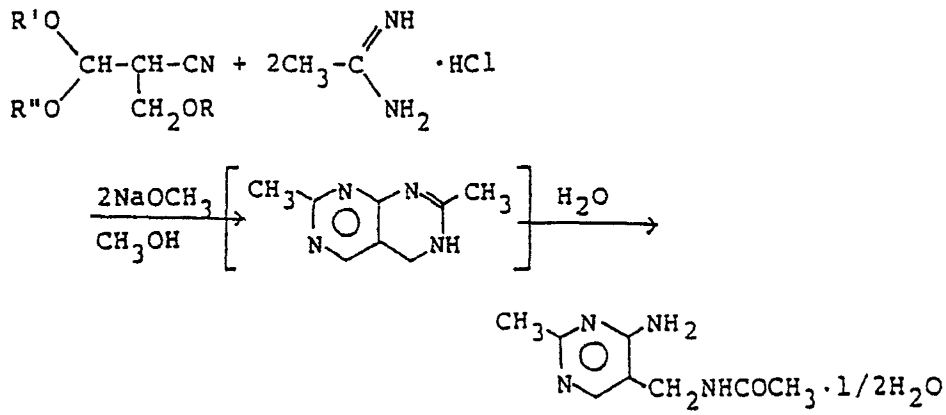


The reaction according to DE—C—937 057 may be summarized as follows:

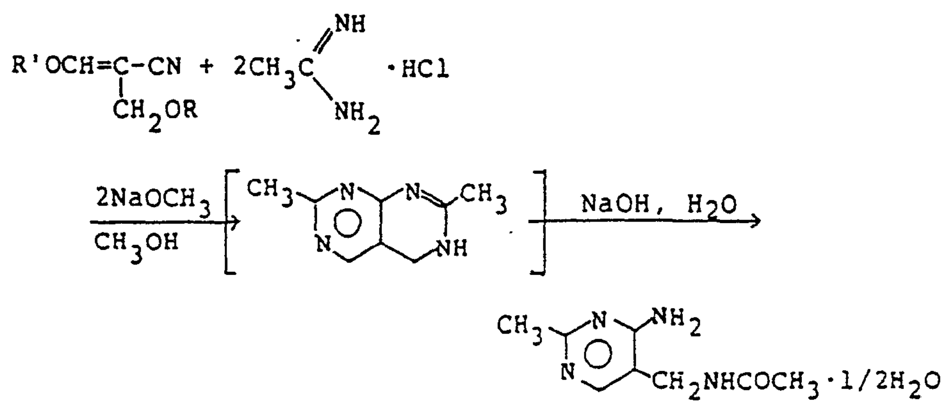


On the other hand, the cyclization reaction according to the present invention may be summarized as follows:

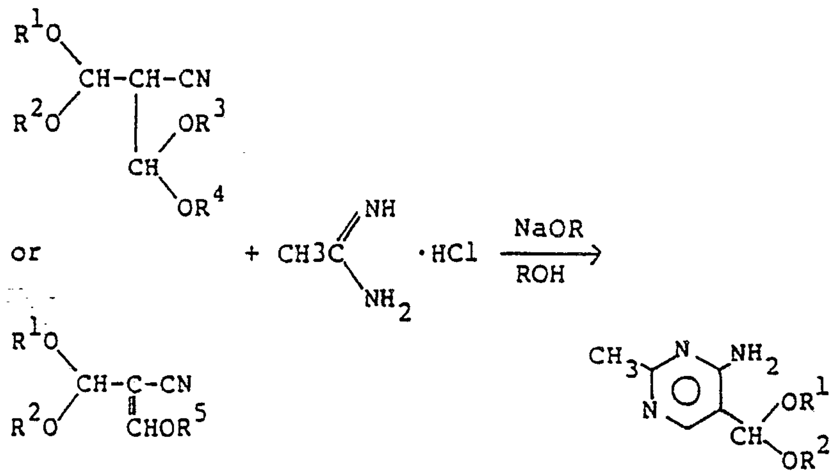


Between the process of the present invention and the processes of the two above mentioned references DE—B—1 016 266 and DE—C—937 057, there are three different points as follows.

1. Although all the processes employ β-aldehydenitrile derivatives, the compound of prior art contains an alkoxymethyl group on the α-carbon relative to the nitrile group, whereas the corresponding compound of the present invention contains an aldehyde group which is higher in the state of oxidation as compared with the alkoxymethyl group in the prior art references.

2. In the process of the present invention, *an equimolar amount* of the amidine compound is sufficient. In the processes of the two prior art references, *2 moles* of the amidine is necessary relative to one mole of the β-aldehydenitrile in view of the reaction mechanism.

3. The yield of the desired product obtained by the process according to the present invention is higher by at least 10 to 20% than those of the prior art processes.

As the result, the present inventors found that, when a propanenitrile derivative represented by the general formula (I) or (II)

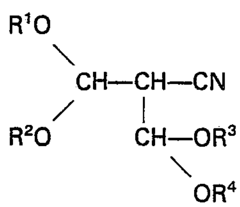

(I)

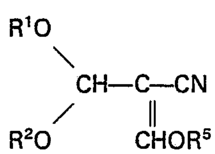

(II)

wherein $R^1$, $R^2$, $R^3$, $R^4$ and $R^5$ may be the same or different and each represent a $C_1$ to $C_5$ alkyl group; or $R^1$, $R^2$, $R^3$ and $R^4$ may be $C_1$ to $C_5$ alkylene groups which are bonded to each other to form a ring or rings, is reacted with an amidine represented by the general formula (III)

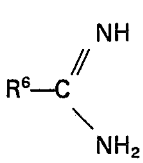

(III)

wherein $R^6$ represents a hydrogen atom, a $C_1$ to $C_5$ alkyl group or a phenyl group of which the hydrogen atom or atoms may be replaced by a $C_1$ to $C_5$ alkyl group, a $C_1$ to $C_5$ alkoxy group or a halogen atom, a 4-amino-5-dialkoxymethylpyrimidine derivative represented by the general formula (IV)

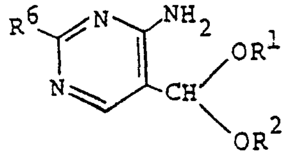

---(IV)

wherein $R^1$, $R^2$ and $R^6$ have the same meanings as defined above, can be prepared with extreme industrial advantage, and accomplished this invention.

As the $R^1$, $R^2$, $R^3$, $R^4$ and $R^5$ in the 2-dialkoxymethyl-3,3-dialkoxypropanenitrile of the above general formula (I) and the 2-alkoxymethylene-3,3-dialkoxypropanenitrile of the above general formula (II) which are the starting materials of this invention and in the 4-amino-5-alkoxymethyl pyrimidine derivatives of the above general formula (IV) which is an object compound, there may be mentioned a lower-alkyl group having 1 to 5 carbon atoms such as methyl, ethyl, propyl and butyl.

Alternatively, $R^1$, $R^2$, $R^3$ and $R^4$ may be lower-alkylene groups having 1 to 5 carbon atoms, which are bonded to each other to form a ring or rings, and there may specifically be mentioned methylene, ethylene, propylene and butylene. These $R^1$, $R^2$, $R^3$, $R^4$ and $R^5$ may all be the same, may partially be the same or may all be different groups.

One of the starting material represented by formula (I) may be prepared in high yield by reacting 2-dichloromethyl-3-chloro-2-propenenitrile, which is a product obtainable by the high temperature vapor phase chlorination of methacrylonitrile, with an alcohol in the presence of a sodium alcoholate [see Yakugaku Zasshi, 1294, *93* (1973) and ibid., 1285, *93* (1973)].

The 2-alkoxymethylene-3,3-dialkoxypropanenitrile (II), is a novel compound and, may be obtained by, for example, reacting a 3-alkoxy-2-propenenitrile or a 3,3-dialkoxypropanenitrile with a formylating agent such as a formic acid ester and carbon monoxide in the presence of an alkali metal alcoholate at a temperature of 0 to 100°C to obtain an alkali metal salt of a 2-hydroxymethylene-3,3-dialkoxypropanenitrile and then reacting the thus obtained salt with an alkylating agent such as a dialkylsulfuric acid and an alkyl halide or with a mineral acid in an amount of not less than an equivalent for neutralization in an alcohol.

A particularly preferred process for preparing the 2-alkoxymethylene-3,3-dialkoxypropanenitrile of formula (II) comprises reacting an alkali metal salt of a 2-hydroxymethylene-3,3-dialkoxypropanenitrile with an alcohol having the formula $R_5$—OH which boils azeotropically with water, in the presence of an acid, while removing said alcohol and produced water from the reaction system by azeotropic distillation.

According to the novel process, the desired 2-alkoxymethylene-3,3-dialkoxypropanenitrile (II) can be prepared in an extremely high yield of around 90%.

Next, the novel process for preparing the 2-alkoxymethylene-3,3-dialkoxypropanenitrile (II) will be described in detail.

The alkali metal salt of the 2-hydroxymethylene-3,3-dialkoxypropanenitrile, which is the starting material for the synthesis of 2-alkoxymethylene-3,3-dialkoxypropanenitrile (II), can be represented by the following general formula (V)

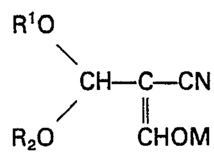

(V)

In the formula, $R^1$ and $R^2$ have the same meanings as defined above and M may include an alkali metal such as sodium, potassium, lithium and rubidium.

As the acid to be used in the process for preparing the compound of formula (II), there may be mentioned an inorganic acid as concentrated sulfuric acid, concentrated hydrochloric acid, hydrogen chloride, and concentrated phosphoric acid, and an organic acid as p-toluenesulfonic acid and acetic acid.

These acids are used in an amount of not less than an equivalent for neutralization, typically 1 to 10 equivalents, preferably 1 to 5 equivalents against the alkali metal salt of the 2-hydroxymethylene-3,3-dialkoxypropanenitrile.

As the representative examples of the alcohol $R^5$—OH which evaporates azeotropically with water, there may be mentioned ethanol, propanols, butanols and pentanols. Above all, n-butanol is most preferable. These alcohols may be employed typically in an amount of 3 to 200 parts by weight, preferably in an amount of 5 to 100 parts by weight per one part by weight of the alkali metal salt of the 2-hydroxymethylene-3,3-dialkoxypropanenitrile, since the progress of the reaction becomes insufficient when the amount to be used thereof is too small and thus the yield of the desired product is decreased, and since the use of larger amount thereof is less economical although any excess amount thereof does not negatively affect the reaction.

The reaction can be carried out under ambient or reduced pressure at a temperature of 0 to 120°C for 1 to 10 hours while removing the formed water from the reaction system by azeotropic distillation with the

used lower-alcohol. In cases where the reaction is not conducted while removing the water from the reaction system, the yield of the desired product becomes extremely low.

In the process for preparing the compound of formula (II) a solvent is not necessarily needed. However, the azeotropic evaporation of the water formed by the reaction may be promoted by using a solvent inert to the reaction which forms an azeotropic mixture of a ternary system with water and the lower-alcohol. As such solvents, there may be mentioned hydrocarbon group solvents such as benzene, toluene, hexane, heptane and cyclohexane; and halogenated hydrocarbon group solvents such as carbon tetrachloride, methylene chloride, ethylene dichloride, trichloroethylene and tetrachloroethylene.

The thus formed products, 2-alkoxymethylene-3,3-dialkoxypropanenitriles (II) may be reacted as such, without isolation thereof, with amidines in the next step to obtain the desired end products.

The products, 2-alkoxymethylene-3,3-dialkoxypropanenitriles (II) may readily be isolated and purified by optionally adopting a procedure or procedures such as filtration, neutralization, extraction, distillation and so forth. The thus isolated products may also be used as the starting material for obtaining the final desired product.

The starting material of formula (I) may also be obtained easily by reacting a 2-alkoxymethylene-3,3-dialkoxypropanenitrile (II) with a corresponding aliphatic alcohol in the presence of an alkali metal alcoholate corresponding to the alkoxy group to be introduced conveniently at a temperature of 0 to 150°C.

As the alcohol to be used, there may be mentioned methanol, ethanol, propanol and butanol.

The amount of the alcohol to be used is in the range of 10 to 500 moles per one mole of the 2-alkoxymethylene-3,3-dialkoxypropanenitrile (II).

As the alkali metal to be used for the alcoholate, there may be mentioned sodium and potassium. The amount of the alcoholate to be used is in the range of 0.05 to 5 moles per one mole of the starting compound (II). Further, the above-mentioned reaction may be carried out in an inert solvent, for example, an ether group solvent, such as tetrahydrofuran, dioxane, dimethoxyethane, diethyl ether and diisopropyl ether, and a hydrocarbon group solvent such as benzene, toluene, xylene, hexane and heptane.

The isolation of the thus formed compound (II) may readily be carried out by optionally adopting such a procedure as neutralization, filtration, extraction, concentration, distillation and so on.

In the present invention, the above-mentioned starting propanenitriles represented by the above general formula (I) or (II) may either be employed alone or may be used as a mixture.

Further, as the amidine represented by the above general formula (III) which is the other starting material, there may be mentioned, for example, formamidine, acetamidine, propioamidine, butanoamidine, pentanoamidine, benzamidine, toluamidine, ethylbenzamidine, propylbenzamidine, methoxybenzamidine, ethoxybenzamidine, chlorobenzamidine and bromobenzamidine. Since these amidines are unstable compounds, it is preferred to use them in the form of a salt with an inorganic acid such as hydrochloric acid, sulfuric acid and nitric acid or with an inorganic acid such as acetic acid. As a base to be used for obtaining a free amidine in the reaction system, there may be mentioned a sodium alcoholate, an alkali hydroxide, an alkali carbonate, an alkali bicarbonate, a strongly basic ion exchange resin and so on. The amidine salt may be used in an amount of 0.5 to 10 moles, preferably 1 to 5 moles per one mole of the propanenitrile represented by the above general formula (I) or (II). The above-mentioned base is employed in an amount of about one equivalent for neutralization.

The reaction may be carried out without a solvent or may be carried out in a solvent which is inert to the reaction. As such solvents, an aliphatic alcohol such as methanol, ethanol, propanol and butanol is most preferable. However, ether group solvents such as dioxane, tetrahydrofuran, dimethoxyethane, diethyl ether, diisopropyl ether and dibutyl ether; aromatic hydrocarbon group solvents such as benzene, toluene and xylene; halogenated hydrocarbon group solvents such as methylene chloride, chloroform, carbon tetrachloride and 1,2-dichloroethane, nitrile group solvents such as acetonitrile, propionitrile and benzonitrile; may also be used for the reaction. These solvents may preferably be employed in an amount of 0.5 to 20 parts by weight per one part by weight of the compound represented by the general formula (I) or (II) (in case of a mixture, per one part of the total amount thereof).

The reaction is conveniently carried out at a temperature of 0 to 150°C under ambient pressure or under positive pressure for 0.1 to 24 hours. The reaction may be carried out either by a batch system or by a continuous system. The isolation of the desired product from the reaction mixture may readily be conducted by optionally adopting a procedure such as filtration, concentration, extraction, recrystallization and so forth.

According to the process of this invention, the 4-amino-5-dialkoxymethylpyrimidine derivative represented by the above general formula (IV) can be prepared in a more simplified method as compared with the processes known to the art.

The 4-amino-5-dialkoxymethylpyrimidine derivative represented by the above general formula (IV) obtained by the process of this invention may readily be converted into a 4-amino-5-aminomethylpyrimidine derivative, which is an important compound as an intermediate for the synthesis of Vitamin-$B_1$, by, for example, hydrolysis thereof in the presence of an acid followed by reductive amination of the thus obtained 4-amino-5-formylpyrimidine derivative.

Example 1

In a 300 ml four-necked flask equipped with a stirrer, a dropping funnel, a thermometer and a

condenser arranged downwardly, there were introduced 8.25 g (50 mmoles) of sodium salt of 2-hydroxymethylene-3,3-dimethoxypropanenitrile and 160 g of n-butanol. Then, with stirring the mixture at room temperature, 3.06 g (30 mmoles) of conc. sulfuric acid was gradually added dropwise thereto. After stirring for one hour, the mixture was heated under a reduced pressure of 30.60 to 33.25 mbar (23 to 25 mmHg) and the temperature of the liquid was maintained at 43 to 45°C to distil out such lower-boiling fractions as alcohol and water. After 1.5 hours from the starting of the distillation, 120 g of n-butanol were further added dropwise thereto, and the reaction was continued for further 2 hours while distilling out the lower-boiling fractions under the same temperature and the same pressure as in the above-mentioned conditions to obtain 170 g in total of a distillate.

After cooling, the reaction mixture was subjected to filtration to remove the insoluble inorganic salt. Thereafter, the filtrate was concentrated followed by distillation under reduced pressure to remove the low-boiling fraction to obtain 127 g (yield: 90%) of a colorless oil boiling at 153 to 156°C/0.66 mbar. (0.5 mmHg).

The thus obtained product was confirmed to be 2-n-butoxymethylene-3,3-di-n-butoxypropanenitrile from the analyses by NMR, IR and MS.

Example 2

Into the same apparatus as in Example 1, were introduced 8.25 g (50 mmoles) of sodium salt of 2-hydroxymethylene-3,3-dimethoxypropanenitrile and 120 g of n-butanol. With stirring the mixture at room temperature, 80 g (hydrogen chloride 100 mmoles) of a 4.6 wt% solution of hydrogen chloride in n-butanol was gradually added. After stirring for one hour, the mixture was heated under a reduced pressure of 30.60 to 33.25 mbar (23 to 25 mmHg) and the temperature of the liquid was maintained at 43 to 45°C to distil out such a low-boiling fraction as alcohol and water. After starting the distillation, the reaction was continued for 2 hours to obtain 130 g of a distillate.

After cooling, the reaction mixture was subjected to gas chromatographic analysis according to the internal standard method for quantitative determination. As a result, it was confirmed that 2-n-butoxymethylene-3,3-di-n-butoxypropanenitrile was produced in a yield of 92%.

Example 3

Into the same apparatus as in Example 1, were introduced 7.47 g (30 mmoles) of sodium salt of 2-hydroxymethylene-3,3-di-n-butoxypropanenitrile and 40 g of n-butanol. With stirring at room temperature, 1.73 g (17 mmoles) of conc. sulfuric acid was gradually added thereto. After stirring for one hour, the mixture was heated under a reduced pressure of 43.9 to 46.7 mbar (33 to 35 mmHg) and the temperature of the liquid was maintained at 50 to 52°C to distil out such a low-boiling fraction as n-butanol and water. After starting of the distillation, 160 g of n-butanol was added dropwise thereto over 3 hours while maintaining the temperature and pressure at the same level as in the above and the reaction was continued further for 0.5 hour to obtain 160 g in total of a distillate.

After cooling, the reaction mixture was subjected to quantitative analysis in the same manner as in Example 2. As a result, it was confirmed that 2-n-butoxymethylene-3,3-di-n-butoxypropanenitrile was produced in a yield of 93%.

Example 4

Into the same apparatus as in Example 1, were introduced 3.86 g (20 mmoles) of sodium salt of 2-hydroxymethylene-3,3-diethoxypropanenitrile, 100 g of ethanol and 100 g of n-hexane. With stirring at room temperature, 1.22 g (12 mmoles) of conc. sulfuric acid were gradually added dropwise thereto. After stirring for one hour, the mixture was heated under ordinary pressure and such a low-boiling fraction as ethanol, n-hexane and water was distilled out at a distillation temperature of 56 to 58°C. After starting of the distillation, a mixture of 250 g of ethanol and 900 g of n-hexane was added dropwise thereto over six hours while maintaining the distillation temperature at the same level and the reaction was further conducted for one hour to obtain 1250 g in total of a distillate.

Subsequently, the reaction mixture was treated in the same manner as in Example 1 to obtain 3.58 g (yield: 90%) of a colorless transparent oil boiling at 120 to 123°C/2.66 mbar (2 mmHg). The thus obtained product was confirmed to be 2-ethoxymethylene-3,3-diethoxypropanenitrile according to the analyses by NMR, IR and MS.

Example 5

Into the same apparatus as in Example 1, were introduced 7.47 g (30 mmoles) of sodium salt of 2-hydroxymethylene-3,3-di-n-butoxypropanenitrile and 200 g of n-butanol. With stirring the mixture at room temperature, 1.73 g (17 mmoles) of conc. sulfuric acid was gradually added dropwise thereto. After stirring for one hour, the temperature of the liquid was maintained at 50 to 52°C and the reaction was carried out for 3.5 hours.

After cooling, the reaction mixture was subjected to quantitative analysis in the same manner as in Example 2 to confirm that 2-n-butoxymethylene-3,3-di-n-butoxypropanenitrile was produced in a yield of 57%.

Example 6

In a 300 ml autoclave made of stainless steel, there were placed 16.6 g (200 mmoles) of 3-methoxy-2-propenenitrile, 13.0 g (240 mmoles) of sodium methylate, 12.8 g (400 moles) of methanol and 65 ml of toluene. After the atmosphere of the reaction system was replaced by nitrogen, the mixture was heated up to around 40°C under stirring and CO was pressured into the autoclave so that the pressure was about 51 bar (50 kg/cm$^2$G). The consumed CO was supplemented continuously and the reaction was continued for 3 hours. After cooling of the reaction mixture, the gas in the autoclave was purged off and the reaction mixture was transferred completely into a 300 ml four-necked flask equipped with a calcium chloride tube, a stirrer, a dropping funnel and a thermometer. While maintaining the liquid temperature at about 20°C or less, 30.3 g (240 mmoles) of dimethylsulfuric acid were added dropwise thereto over 30 minutes with stirring the contents in the flask, and the reaction was carried out at around 50°C for about 4 hours.

After the reaction mixture was cooled and the insoluble substance was removed by filtration, the collected insoluble were washed with toluene and the washing was combined with the filtrate followed by washing with a 50 wt% aqueous sodium hydroxide and then with water. Subsequently, after drying over sodium sulfate, the toluene layer was evaporated under reduced pressure to remove low-boiling fractions and to obtain 17.6 g (yield: 56%) of a colorless transparent oil boiling at 104—106°C/2.66 mbar (2 mmHg).

The thus obtained product was confirmed to be 2-methoxymethylene-3,3-dimethoxypropanenitrile according to analyses by NMR, IR and MS.

Example 7

In a 200 ml flask equipped with a condenser arranged downwardly, there were placed 50.0 g (0.032 mole) of 2-methoxymethylene-3,3-dimethoxypropanenitrile and 20.0 g (0.32 mole) of ethylene glycol. Then, the mixture was heated on a bath, which was maintained at 100°C, for 2 hours under ambient pressure to distil off methanol. Subsequently, the resulting mixture was evaporated under reduced pressure to remove a fraction boiling at low temperatures, followed by fractionation to give 33.0 g (yield: 67%) of a colorless transparent oil boiling at 133—135°C/2.66 mbar (2 mmHg). The thus obtained product was confirmed to be methoxymethylenecyanoacetaldehyde ethylene acetal according to analyses by NMR, IR and MS.

Example 8

In a mixed solvent of 100 ml of toluene and 50 ml of methanol was dissolved 7.85 g (50 mmoles) of 2-methoxymethylene-3,3-dimethoxypropanenitrile. To the resulting solution were added 9.65 g (50 mmoles) of a 28 wt% solution of sodium methylate in methanol and the mixture was stirred at room temperature for one and a half hours. Subsequently, the reaction mixture was concentrated under reduced pressure to remove the methanol by distillation. To the resulting residue was added 25 ml of water and the toluene layer was separated followed by drying over anhydrous sodium sulfate. The thus dried toluene solution was distilled to obtain 8.50 g (45 mmoles) of 2-dimethoxymethyl-3,3-dimethoxypropanenitrile as a colorless transparent oil boiling at 90—93°C/2.66 mbar (2 mmHg).

Example 9

In 100 ml of n-butanol were dissolved 8.49 g (30 mmoles) of 2-n-butoxymethylene-3,3-di-n-butoxypropanenitrile. To the resulting solution were added 0.87 g (9 mmoles) of sodium n-butylate at room temperature, and the mixture was stirred at room temperature for one hour. Then, the reaction mixture was neutralized with a solution of sulfuric acid in butanol and the resulting inorganic salt was removed by filtration. The filtrate was distilled under reduced pressure to obtain 9.10 g (25 mmoles) of 2-di-n-butoxymethyl-3,3-di-n-butoxypropanenitrile as a colorless transparent oil boiling at 170—175°C/2.66 mbar (2 mmHg).

In the following Examples, the starting materials have been obtained by one of the processes as described above in Examples 1 to 6.

Example 10

In a 50 ml four-necked flask equipped with a calcium-chloride tube, a thermometer and a reflux condenser, there were placed 11.6 g (60 mmoles) of a 28 wt% solution of sodium methylate in methanol and 10 ml of methanol. Thereto were added with stirring 5.67 of acetamidine hydrochloride (60 mmoles) and the mixture was stirred at room temperature for 30 minutes. Then, 7.85 g (50 mmoles) of 2-methoxymethylene-3,3-dimethoxypropanenitrile were added thereto and the mixture was heated and refluxed for 5 hours. After completion of the reaction, the reaction mixture was cooled and then the insoluble sodium chloride was removed by filtration. After concentration of the solvent, methanol, 50 ml of water was added thereto and the mixture was extracted four times with 20 ml of methylene chloride. After the extract was dried over sodium sulfate, the sodium sulfate was removed by filtration and the filtrate was concentrated to dryness to obtain a white crude crystal. The thus obtained product was recrystallized from a mixed solvent of 50 ml of hexane and 25 ml of toluene to obtain 8.05 g (yield: 88%) of 2-methyl-4-amino-5-dimethoxymethylpyrimidine melting at 102—104°C.

Example 11

In the same reaction apparatus as in Example 10, there were placed 11.6 g (60 mmoles) of a 28 wt% solution of sodium methylate in methanol. Thereto were added with stirring 5.67 g (60 mmoles) of acetamidine hydrochloride, and the mixture was stirred at room temperature for one hour. Subsequently, 9.45 g (50 mmoles) of 2-dimethoxymethyl-3,3-dimethoxypropanenitrile were added thereto and the mixture was heated and refluxed for 5 hours. After completion of the reaction, the reaction mixture was cooled and the insoluble sodium chloride was removed by filtration. The filtrate was analyzed by gas chromatography according to the internal standard method. As the result, it was confirmed that 8.51 g (yield: 93%) of 2-methyl-4-amino-5-dimethoxymethylpyrimidine had been formed.

Example 12

An experiment was carried out in the same manner as in Example 11 except that a mixture containing 3.31 g of 2-dimethoxymethyl-3,3-dimethoxypropanenitrile (17.5 mmoles) and 5.10 g (32.5 mmoles) of 2-methoxymethylene-3,3-dimethoxypropanenitrile was used in place of the 2-dimethoxymethyl-3,3-dimethoxypropanenitrile. The yield of 2-methyl-4-amino-5-dimethoxymethylpyrimidine was 91%.

Example 13

In the same apparatus as in Example 10, there were placed 2.84 g (30 mmoles) of acetamidine hydrochloride and 15 ml of ethanol. Thereto was added with stirring 2.04 g (30 mmoles) of sodium ethylate and the mixture was stirred at room temperature for 30 minutes. Subsequently, a mixture containing 2.45 g (10 mmoles) of 2-diethoxymethyl-3,3-diethoxypropanenitrile and 2.99 g (15 mmoles) of 2-ethoxymethylene-3,3-diethoxypropanenitrile was added thereto and the mixture was heated followed by reflux for 4 hours. After cooling, sodium chloride was removed and the filtrate was subjected to gas chromatographic analysis. The yield of 2-methyl-4-amino-5-diethoxymethylpyrimidine was 88%.

Example 14

In the same apparatus as in Example 10, 2.84 g (30 mmoles) of acetoamidine hydrochloride was added to a solution of sodium butylate in n-butanol which had been prepared with 0.69 g (30 mg atoms) of sodium and 15 ml of n-butanol, and the mixture was stirred at room temperature for 30 minutes. Then, 7.08 g (25 mmoles) of 2-n-butoxymethylene-3,3-di-n-butoxypropanenitrile were added thereto and the mixture was heated. Thereafter, the resulting mixture was stirred under heating at about 90°C for 5 hours. After cooling, the sodium chloride was removed and the remaining liquid was subjected to gas chromatographic analysis for quantitative determination. The yield of 2-methyl-4-amino-5-di-n-butoxymethylpyrimidine was 85%.

Example 15

An experiment was conducted in the same manner as in Example 10 except that 9.40 g (60 mmoles) of benzamidine hydrochloride was used in place of the acetamidine hydrochloride to obtain white crude crystals. These crystals thus obtained were recrystallized from a mixed solvent of hexane and toluene (volume ratio 2:1) to obtain 10.4 g (yield: 85%) of 2-phenyl-4-amino-5-dimethoxymethylpyrimidine melting at 116—118°C.

Example 16

An experiment was carried out in the same manner as in Example 11 except that 7.75 g (50 mmoles) of methoxymethylenecyanoacetaldehyde ethylene acetal were used in place of 2-dimethoxymethyl-3,3-dimethoxypropanenitrile.

The yield of ethylene acetal of 2-methyl-4-amino-5-formylpyrimidine was 83%.

Example 17

An experiment was conducted in the same manner as in Example 11 except that 4.83 g (60 mmoles) of formamidine hydrochloride were used in place of acetamidine hydrochloride. The yield of 4-amino-5-dimethoxymethylpyrimidine was 90%.

Example 18

An experiment was conducted in the same manner as in Example 11 except that 6.51 g (60 mmoles) of propioamidine hydrochloride were used in place of acetamidine hydrochloride.

The yield of 2-ethyl-4-amino-5-dimethoxymethylpyrimidine was 89%.

Example 19

In a 200 ml four-necked flask equipped with a stirrer, a dropping funnel, a thermometer and a condenser arranged downwardly, there were introduced 8.25 g (50 mmoles) of sodium salt of 2-hydroxymethylene-3,3-dimethoxypropanenitrile and 60 g of n-butanol. To the mixture were gradually added dropwise, with stirring, 2.76 g (27 mmoles) of conc. sulfuric acid. After stirring for one hour, the temperature of the mixture was raised under a reduced pressure of 43.9 to 46.7 mbar (33—35 mmHg), and then was maintained at 50 to 52°C to distill off a low-boiling fraction of the alcohol and water. After starting the distillation, 160 g of n-butanol were added dropwise thereto over 2 hours while maintaining the

temperature and the pressure at the same level, and the reaction was further continued for 30 minutes to obtain 160 g in total of a distillate after the heating was stopped and the reaction system was returned to an ordinary pressure. Subsequently, the downwardly arranged condenser was replaced with a reflux condenser equipped with a calcium chloride tube. Then, a mixture of 5.20 g (55 mmoles) of acetamidine hydrochloride and 30.0 g (62.5 mmoles) of a 20 wt% solution of sodium n-butylate in n-butanol, which had been stirred under reduced pressure in a dry atmosphere for 30 minutes, was gradually added dropwise, with stirring, to the flask from the dropping funnel.

Thereafter, the temperature of the mixture was raised and the resulting mixture was stirred under heating at 85 to 90°C for around 4 hours.

After cooling, sodium chloride was removed by filtration and the filtrate thus obtained were subjected to quantitative analysis by gas chromatography. As the result, it was found that 11.0 g (41.2 mmoles) of 2-methyl-4-amino-5-di-n-butoxymethylpyrimidine was obtained. The yield of the product is 82% based on the starting material, i.e., sodium salt of 2-hydroxymethylene-3,3-dimethoxypropanenitrile.

## Claims

1. A process for preparing a 4-amino-5-dialkoxymethylpyrimidine derivative represented by the formula

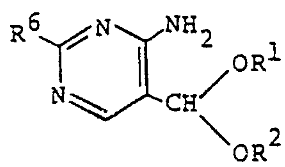

wherein $R^1$ and $R^2$ may be the same or different and each represent a $C_1$ to $C_5$-alkyl group, or $R^1$ and $R^2$ may be lower-alkylene groups which are bonded to each other to form a ring; and $R^6$ represents a hydrogen atom, a $C_1$ to $C_5$-alkyl group or a phenyl group of which the hydrogen atom or atoms may be replaced by a $C_1$ to $C_5$-alkyl group, a $C_1$ to $C_5$-alkoxy group or a halogen atom, which comprises subjecting a propanenitrile derivative represented by the formula (I) or (II)

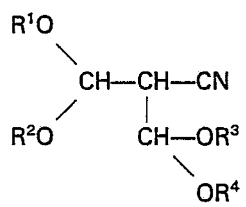

(I)

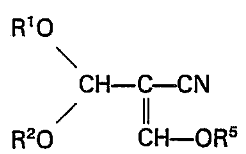

(II)

wherein $R^1$, $R^2$, $R^3$, $R^4$ and $R^5$ may be the same or different and each represent a $C_1$ to $C_5$ alkyl group; or $R^1$, $R^2$, $R^3$ and $R^4$ may be $C_1$ to $C_5$-alkylene groups which are bonded to each other to form a ring or rings, to reaction with an amidine represented by the formula

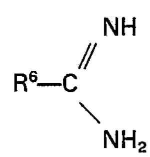

(III)

wherein $R^6$ has the same meaning as defined above.

2. A process according to Claim 1, wherein $R^1$, $R^2$, $R^3$, $R^4$, $R^5$ and $R^6$ in the formulas represent an alkyl group selected from methyl, ethyl, propyl and butyl.

3. A process according to Claim 1, wherein the amidine is selected from formamidine, acetamidine, propioamidine, butanoamidine, pentanoamidine, benzamidine, toluamidine, ethylbenzamidine,

propylbenzamidine, methoxybenzamidine, ethoxybenzamidine, chlorobenzamidine and bromobenzamidine.

4. A process according to Claim 1, wherein the amidine is used in the form of a salt with an inorganic or organic acid and is then converted into a free form in the reaction system.

5. A process according to Claim 1, wherein the amidine is used in an amount of 0.5 to 10 moles per one mole of the propanenitrile represented by the formula (I) or (II).

6. A process according to Claim 5, wherein the amidine is used in an amount of 1 to 5 moles per mole of the propanenitrile represented by the formula (I) or (II).

7. A process according to Claim 1, wherein the reaction is carried out at a temperature of 0 to 150°C under ambient or positive pressure for 0.1 to 24 hours.

8. A process according to Claim 1, wherein the reaction is carried out in an inert solvent.

9. A process according to Claim 8, wherein the amount of the solvent to be used is in the range of 0.5 to 20 parts by weight per one part by weight of the compound represented by the formula (I) or (II).

10. A process according to Claim 1, wherein the propanenitrile represented by the formula (II) has been prepared by reacting an alkali metal salt of a 2-hydroxymethylene-3,3-dialkoxypropanenitrile with an alcohol having the formula $R^5$—OH in which $R^5$ has the same meaning as in claim 1, which boils azeotropically with water, in the presence of an acid, while removing said alcohol and produced water from the reaction system by azeotropic distillation.

11. A process according to Claim 10, wherein the alkali metal is selected from sodium, potassium, lithium and rubidium.

12. A process according to Claim 10, wherein the acid is selected from conc. sulfuric acid, conc. hydrochloric acid, hydrogen chloride, conc. phosphoric acid, p-toluenesulfonic acid and acetic acid.

13. A process according to Claim 10, wherein the amount of the acid to be used is in the range of 1 to 10 equivalents against the alkali metal salt of the 2-hydroxymethylene-3,3-dialkoxypropanenitrile.

14. A process according to Claim 13, wherein the amount of the acid to be used is in the range of 1 to 5 equivalents against the alkali metal salt of the 2-hydroxymethylene-3,3-dialkoxypropanenitrile.

15. A process according to Claim 10, wherein the alcohol is selected from ethanol, propanol, butanol and pentanol.

16. A process according to Claim 10, wherein the amount of the alcohol to be used is in the range of 3 to 200 parts by weight per one part by weight of the alkali metal salt of the 2-hydroxymethylene-3,3-dialkoxypropanenitrile.

17. A process according to Claim 16, wherein the amount of the alcohol to be used in the range of 5 to 100 parts by weight per one part by weight of the alkali metal salt of the 2-hydroxymethylene-3,3-dialkoxypropanenitrile.

18. A process according to Claim 10, wherein the reaction for forming the propanenitrile represented by formula (II) is carried out under ambient or reduced pressure at a temperature of 0 to 120°C for 1 to 10 hours.

19. A process according to Claim 1, wherein the propanenitrile represented by the formula (I) has been prepared by reacting a 2-alkoxymethylene-3,3-dialkoxypropanenitrile with a corresponding alcohol in the presence of an alkali metal alcoholate corresponding to the alkoxy group to be introduced.

20. A process according to Claim 19, wherein the reaction is carried out at a temperature of 0 to 150°C.

21. A process according to Claim 20, wherein said alcohol is selected from methanol, ethanol, propanol and butanol.

22. A process according to Claim 19, wherein the amount of the alcohol to be used is in the range of 10 to 500 moles per one mole of the 2-alkoxymethylene-3,3-dialkoxypropanenitrile.

23. A process according to Claim 19, wherein said alkali metal is sodium or potassium.

24. A process according to Claim 19, wherein said alcoholate is used in an amount of 0.05 to 5 moles per one mole of the 2-alkoxymethylene-3,3-dialkoxypropanenitrile.

25. A compound represented by the general formula:

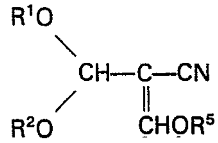

wherein $R^1$, $R^2$ and $R^5$ may be the same or different and each represent a $C_1$ to $C_5$ alkyl group, or $R^1$ and $R^2$, bonding to each other to form a ring, are a $C_1$ to $C_5$-alkylene group.

26. A compound of Claim 25, wherein $R^1$, $R^2$ and $R^5$ are n-butyl, respectively.

27. A process for preparing a compound represented by the formula (II)

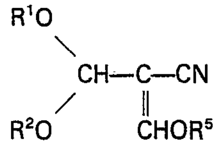

(II)

wherein $R^1$, $R^2$ and $R^5$ may be the same or different and each represent a $C_1$ to $C_5$-alkyl group, or $R^1$ and $R^2$, bonding to each other to form a ring, are a $C_1$ to $C_5$-alkylene group, which comprises reacting a compound represented by the formula (V)

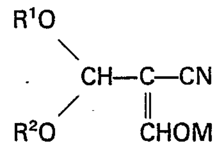

(V)

wherein $R^1$ and $R^2$ have the same meanings as defined above, and M represents an alkali metal, with an alcohol of formula $R^5$—OH in which $R^5$ has the above meaning, which boils azeotropically with water, in the presence of an acid, while removing said alcohol and produced water from the reaction system by azeotropic distillation.

28. A process according to Claim 27, wherein the alkali metal is selected from sodium, potassium, lithium and rubidium.

29. A process according to Claim 27, wherein the acid is selected from conc. sulfuric acid, conc. hydrochloric acid, hydrogen chloride, conc. phosphoric acid, p-toluenesulfonic acid and acetic acid.

30. A process according to Claim 27, wherein the amount of the acid to be used is in the range of 1 to 10 equivalents against the compound represented by formula (V).

31. A process according to Claim 27, wherein the alcohol is selected from ethanol, propanols, butanols and pentanols.

32. A process according to Claim 31, wherein the alcohol is n-butanol.

33. A process according to Claim 27, wherein the amount of the alcohol to be used is in the range of 3 to 200 parts by weight per one part by weight of the compound represented by formula (V).

34. A process according to Claim 27, wherein the reaction for forming the compound represented by formula (II) is carried out under ambient or reduced pressure at a temperature of 0 to 120°C.

**Patentansprüche**

1. Verfahren zur Herstellung eines 4-Amino-5-dialkoxymethylpyrimidin-derivats der Formel

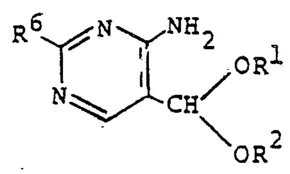

worin $R^1$ und $R^2$ gleich oder verschieden sein können, jeweils eine $C_{1-5}$-Alkylgruppe bedeuten oder $R^1$ und $R^2$ Niedrigalkylengruppen sein können, die unter Ausbildung eines Ringes miteinander verbunden sind und $R^6$ ein Wasserstoffatom, eine $C_{1-5}$-Alkylgruppe oder eine Phenylgruppe, bei welcher das Wasserstoffatom oder die Wasserstoffatome durch eine $C_{1-5}$-Alkylgruppe ersetzt sein können, eine $C_{1-5}$-Alkoxygruppe oder ein Halogenatom bedeutet, dadurch gekennzeichnet, dass man ein Propannitrilderivat der allgemeinen Formeln (I) oder (II)

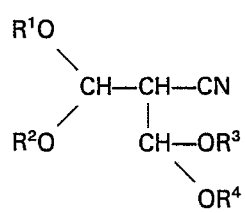

(I)

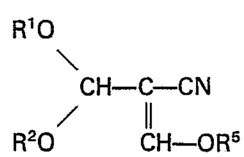

(II)

worin $R^1$, $R^2$, $R^3$, $R^4$ und $R^5$ gleich oder verschieden sein können und jeweils eine $C_{1-5}$-Alkylgruppe bedeuten; oder $R^1$, $R^2$, $R^3$ und $R^4$ $C_{1-5}$-Alkylengruppen sein können, die miteinander unter Ausbildung eines Ringes oder von Ringen verbunden sind, mit einem Amidin der Formel

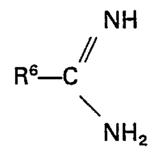

in welcher $R^6$ die vorher angegebene Bedeutung hat, umsetzt.

2. Verfahren gemäss Anspruch 1, bei dem $R^1$, $R^2$, $R^3$, $R^4$, $R^5$ und $R^6$ in den Formeln Alkylgruppen, ausgewählt aus Methyl, Ethyl, Propyl und Butyl, bedeuten.

3. Verfahren gemäss Anspruch 1, bei dem das Amidin ausgewählt ist aus Formamidin, Acetamidin, Propioamidin, Butanoamidin, Pentanoamidin, Benzamidin, Toluamidin, Ethylbenzamidin, Propylbenzamidin, Methoxybenzamidin, Ethoxybenzamidin, Chlorbenzamidin und Bromdbenzamidin.

4. Verfahren gemäss Anspruch 1, bei dem das Amidin in Form eines Salzes mit einer anorganischen oder organischen Säure verwendet wird und in dem Reaktionssystem dann in die freie Form umgewandelt wird.

5. Verfahren gemäss Anspruch 1, dadurch gekennzeichnet, dass das Amidin in einer Menge von 0,5 bis 10 Molen pro 1 Mol des durch die Formeln (I) oder (II) bezeichneten Propannitrils verwendet wird.

6. Verfahren gemäss Anspruch 5, bei dem das Amidin in einer Menge von 1 bis 5 Molen pro 1 Mol des durch die Formeln (I) oder (II) dargestellten Propannitrils verwendet wird.

7. Verfahren gemäss Anspruch 1, bei dem die Umsetzung bei einer Temperatur von 0 bis 150°C unter Umgebungsdruck oder einem positiven Druck während 0,1 bis 24 Stunden durchgeführt wird.

8. Verfahren gemäss Anspruch 1, bei dem man die Umsetzung in einem inerten Lösungsmittel durchführt.

9. Verfahren gemäss Anspruch 8, bei dem die Menge des zu verwendenden Lösungsmittels im Bereich von 0,5 bis 20 Gew.-Teilen pro 1 Gew.-Teil der durch die Formeln (I) oder (II) dargestellten Verbindungen beträgt.

10. Verfahren gemäss Anspruch 1, bei dem das durch die Formel (II) dargestellte Propannitril hergestellt wurde durch Umsetzen eines Alkalimetallsalzes eines 2-Hydroxy-methylen-3,3-dialkoxypropannitrils mit einem Alkohol der Formel $R^5$—OH, worin $R^5$ die gleiche Bedeutung wie in Anspruch 1 hat, der mit Wasser azeotrop siedet, in Gegenwart einer Säure, wobei man den Alkohol und das gebildete Wasser aus dem Reaktionssystem durch azeotrope Destillation entfernt.

11. Verfahren gemäss Anspruch 10, bei dem das Alkalimetall ausgewählt ist aus Natrium, Kalium, Lithium and Rubidium.

12. Verfahren gemäss Anspruch 10, bei dem die Säure ausgewählt ist aus konzentrierter Schwefelsäure, konzentrierter Salzsäure, Chlorwasserstoff, konzentrierter Phosphorsäure, p-Toluolsulfonsäure und Essigsäure.

13. Verfahren gemäss Anspruch 10, bei dem die Menge der zu verwendenden Säure im Bereich von 1 bis 10 Äquivalenten, bezogen auf das Alkalisalz des 2-Hydroxymethylen-3,3-dialkoxypropannitrils, liegt.

14. Verfahren gemäss Anspruch 13, bei dem die Menge der zu verwendenden Säure im Bereich von 1 bis 5 Äquivalenten in bezug auf das Alkalisalz des 2-Hydroxymethylen-3,3-dialkoxypropannitrils, liegt.

15. Verfahren gemäss Anspruch 10, bei dem der Alkohol ausgewählt ist aus Ethanol, Propanol, Butanol und Pentanol.

16. Verfahren gemäss Anspruch 10, bei dem die Menge des zu verwendenden Alkohols im Bereich von 3 bis 200 Gew.-Teilen pro 1 Gew.-Teil des Alkalisalzes des 2-Hydroxymethylen-3,3-dialkoxypropannitrils, liegt.

17. Verfahren gemäss Anspruch 16, bei dem die Menge des zu verwendenden Alkohols im Bereich von 5 bis 100 Gew.-Teilen pro 1 Gew.-Teil des Alkalisalzes des 2-Hydroxymethylen-3,3-dialkoxypropannitrils liegt.

18. Verfahren gemäss Anspruch 10, bei dem die Umsetzung zur Bildung des Propannitrils der Formel (II) unter Umgebungs- oder verringertem Druck bei einer Temperatur von 0 bis 120°C während 1 bis 10 Stunden durchgeführt wird.

19. Verfahren gemäss Anspruch 1, bei dem das Propannitril der Formel (I) durch Umsetzen eines 2-Alkoxymethylen-3,3-dialkoxypropannitrils mit einem entsprechenden Alkohol in Gegenwart eines Alkalialkoholats entsprechend der einzuführenden Alkoxygruppe hergestellt wurde.

20. Verfahren gemäss Anspruch 19, bei dem die Umsetzung bei einer Temperatur von 0 bis 150°C durchgeführt wird.

21. Verfahren gemäss Anspruch 20, bei dem der Alkohol aus Methanol, Ethanol, Propanol und Butanol ausgewählt ist.

22. Verfahren gemäss Anspruch 19, bei dem die Menge des zu verwendenden Alkohols im Bereich von 10 bis 500 Molen pro 1 Mol 2-Alkoxymethylen-3,3-dialkoxypropannitril liegt.

23. Verfahren gemäss Anspruch 19, bei dem das Alkalimetall Natrium oder Kalium ist.

24. Verfahren gemäss Anspruch 19, bei dem das Alkoholat in einer Menge von 0,05 bis 5 Molen pro 1 Mol des 2-Alkoxymethylen-3,3-dialkoxypropannitrils verwendet wird.

25. Verbindung der allgemeinen Formel

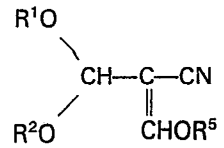

worin $R^1$, $R^2$ und $R^5$, die gleich oder verschieden sein können, jeweils eine $C_{1-5}$-Alkylgruppe bedeuten oder $R^1$ und $R^2$, die unter Ausbildung eines Ringes miteinander verbunden sind, eine $C_{1-5}$-Alkylengruppe sind.

26. Verfahren gemäss Anspruch 25, worin $R^1$, $R^2$ und $R^5$ jeweils n-Butyl sind.

27. Verfahren zur Herstellung einer Verbindung der Formel (II)

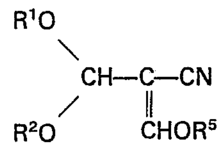

(II)

worin $R^1$, $R^2$ und $R^5$, die gleich oder verschieden sein können, jeweils eine $C_{1-5}$-Alkylgruppe bedeuten oder $R^1$ und $R^2$, die unter Ausbildung eines Ringes miteinander verbunden sind, eine $C_{1-5}$-Alkylengruppe bedeuten, dadurch gekennzeichnet, dass man eine Verbindung der Formel (V)

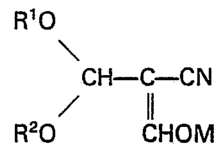

(V)

in welcher $R^1$ und $R^2$ die vorher angegebenen Bedeutungen haben und M ein Alkalimetall bedeutet, mit einem Alkohol der Formel $R^5$—OH, in welcher $R^5$ die vorher angegebene Bedeutung hat, der azeotrop mit Wasser siedet, in Gegenwart einer Säure unter Entfernung des Alkohols und des gebildeten Wassers aus dem Reaktionssystem durch azeotrope Destillation umsetzt.

28. Verfahren gemäss Anspruch 27, bei dem das Alkalimetall ausgewählt ist aus Natrium, Kalium, Lithium and Rubidium.

29. Verfahren gemäss Anspruch 27, bei dem die Säure ausgewählt ist aus konzentrierter Schwefelsäure, konzentrierter Salzsäure, Chlorwasserstoff, konzentrierter Phosphorsäure, p-Toluolsulfonsäure und Essigsäure.

30. Verfahren gemäss Anspruch 27, bei dem die Menge der verwendeten Säure im Bereich von 1 bis 10 Äquivalenten, bezogen auf die Verbindung der Formel (V), liegt.

31. Verfahren gemäss Anspruch 27, bei dem der Alkohol ausgewählt ist aus Ethanol, Propanolen, Butanolen und Pentanolen.

32. Verfahren gemäss Anspruch 31, bei dem der Alkohol n-Butanol ist.

33. Verfahren gemäss Anspruch 27, bei dem die Menge des zu verwendenden Alkohols im Bereich von 3 bis 200 Gew.-Teilen pro 1 Gew.-Teil der Verbindung der Formel (V) liegt.

34. Verfahren gemäss Anspruch 27, bei dem die Umsetzung zur Herstellung einer Verbindung der Formel (II) unter Umgebungs- oder verringertem Druck bei einer Temperatur von 0 bis 120°C durchgeführt wird.

**Revendications**

1. Procédé pour la préparation d'un dérivé 4-amino-5-dialcoxyméthylpyrimidine représenté par la formule

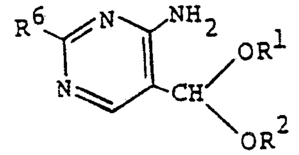

dans laquelle $R^1$ et $R^2$ peuvent être identiques ou différents et représentent chacun un groupe alkyle en $C_1$ à $C_5$, ou $R^1$ et $R^2$ peuvent être des groupes alkylène inférieur qui sont liés l'un à l'autre pour former un noyau; et $R^6$ représente un atome d'hydrogène, un groupe alkyle en $C_1$ à $C_5$ ou un groupe phényle dont un atome ou des atomes d'hydrogène peuvent être remplacés par un groupe alkyle en $C_1$ à $C_5$, un groupe alcoxy en $C_1$ à $C_5$ ou un atome d'halogène, qui comporte le réaction d'un dérivé de propanenitrile représenté par la formule (I) ou (II)

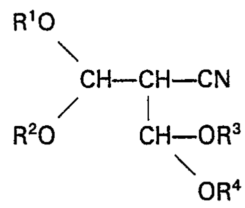

(I)

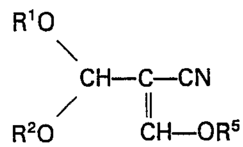

(II)

dans laquelle $R^1$, $R^2$, $R^3$, $R^4$ et $R^5$ peuvent être identiques ou différents et représentent chacun un groupe alkyle en $C_1$ à $C_5$, ou $R^1$, $R^2$, $R^3$ et $R^4$ peuvent être des groupes alkylène en $C_1$ à $C_5$ qui sont liés les uns aux autres pour former un noyau ou des noyaux, avec une amidine représentée par la formule

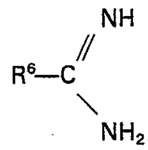

dans laquelle $R^6$ a la même définition que celle donnée ci-dessus.

2. Procédé selon la revendication 1, dans lequel $R^1$, $R^2$, $R^3$, $R^4$, $R^5$ et $R^6$ dans les formules représentent un groupe alkyle choisi parmi les groupes méthyle, éthyle, propyle et butyle.

3. Procédé selon la revendication 1, dans lequel l'amidine est choisi parmi la formamidine, l'acétamidine, la propioamidine, la butanoamidine, la pentanoamidine, la benzamidine, la toluamidine, l'éthylbenzamidine, la propylbenzamidine, la méthoxybenzamidine, l'éthoxybenzamidine, la chlorobenzamidine et la bromobenzamidine.

4. Procédé selon la revendication 1, dans lequel on utilise l'amidine sous la forme d'un sel avec un acide organique ou inorganique et ensuite on la transforme en la forme libre dans le système de réaction.

5. Procédé selon la revendication 1, dans lequel on utilise l'amidine en une quantité de 0,5 à 10 moles pour une mole du propanenitrile représenté par la formule (I) ou (II).

6. Procédé selon la revendication 5, dans lequel on utilise l'amidine en une quantité de 1 à 5 moles pour une mole du propanenitrile représenté par la formule (I) ou (II).

7. Procédé selon la revendication 1, dans lequel on effectue la réaction à une température allant de 0 à 150°C, sous la pression ambiante ou sous une pression positive, pendant de 0,1 à 24 heures.

8. Procédé selon la revendication 1, dans lequel on effectue la réaction dans un solvant inerte.

9. Procédé selon la revendication 8, dans lequel la quantité de solvant à utiliser est comprise dans l'intervalle allant de 0,5 à 20 parties en poids pour une partie en poids du composé représenté par la formule (I) ou (II).

10. Procédé selon la revendication 1, dans lequel on a préparé le propanenitrile représenté par la formule (II) en faisant réagir un sel de métal alcalin d'un 2-hydroxyméthylène-3,3-dialcoxypropanenitrile avec un alcool ayant la formule $R^5$—OH dans laquelle $R^5$ a la même signification que dans la revendication 1 et qui bout en formant un azéotrope avec l'eau, en présence d'un acide, tout en éliminant ledit alcool et l'eau produite dans le système de réaction par distillation azéotropique.

11. Procédé selon la revendication 10, dans lequel le métal alcalin est choisi parmi le sodium, le potassium, le lithium et le rubidium.

12. Procédé selon la revendication 10, dans lequel l'acide est choisi parmi l'acide sulfurique concentré, l'acide chlorhydrique concentré, l'acide chlorhydrique, l'acide phosphorique concentré, l'acide p-toluènesulfonique et l'acide acétique.

13. Procédé selon la revendication 10, dans lequel la quantité d'acide à utiliser est comprise dans la gamme de 1 à 10 équivalents par rapport au sel de métal alcalin du 2-hydroxyméthylène-3,3-dialcoxypropanenitrile.

14. Procédé selon la revendication 13, dans lequel la quantité de l'acide à utiliser est comprise dans la gamme de 1 à 5 équivalents par rapport au sel de métal alcalin du 2-hydroxyméthylène-3,3-dialcoxypropanenitrile.

15. Procédé selon la revendication 10, dans lequel l'alcool est choisi parmi l'éthanol, le propanol, le butanol et le pentanol.

16. Procédé selon la revendication 10, dans lequel la quantité de l'alcool à utiliser est comprise dans l'intervalle allant de 3 à 200 parties en poids pour une partie en poids du sel de métal alcalin du 2-hydroxyméthylène-3,3-dialcoxypropanenitrile.

17. Procédé selon la revendication 16, dans lequel la quantité de l'alcool à utiliser est comprise dans l'intervalle allant de 5 à 100 parties en poids pour une partie en poids du sel de métal alcalin du 2-hydroxyméthylène-3,3-dialcoxypropanenitrile.

18. Procédé selon la revendication 10, dans lequel on effectue la réaction pour former le propanenitrile représenté par la formule (II) sous une pression réduite ou ambiante, à une température allant de 0 à 120°C, pendant 1 à 10 heures.

19. Procédé selon la revendication 1, dans lequel on a préparé le propanenitrile représenté par la formule (I) en faisant réagir un 2-alcoxyméthylène-3,3-dialcoxypropanenitrile avec un alcool correspondant, en présence d'un alcoolate de métal alcalin correspondant au groupe alcoxy à introduire.

20. Procédé selon la revendication 19, dans lequel on effectue la réaction à une température allant de 0 à 150°C.

21. Procédé selon la revendication 20, dans lequel on choisit ledit alcool parmi le méthanol, l'éthanol, le propanol et le butanol.

22. Procédé selon la revendication 19, dans lequel la quantité de l'alcool à utiliser est comprise dans l'intervalle de 10 à 500 moles pour une mole de 2-alcoxyméthylène-3,3-dialcoxypropanenitrile.

23. Procédé selon la revendication 19, dans lequel ledit métal alcalin est le sodium ou le potassium.

24. Procédé selon la revendication 19, dans lequel on utilise ledit alcoolate en une quantité allant de 0,05 à 5 moles pour une mole de 2-alcoxyméthylène-3,3-dialcoxypropanenitrile.

25. Composé représenté par la formule générale

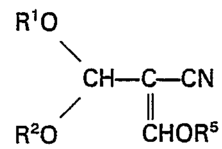

dans laquelle $R^1$, $R^2$ et $R^5$ peuvent être identiques ou différents et représentent chacun un groupe alkyle en $C_1$ à $C_5$, ou $R^1$ et $R^2$, liés l'un à l'autre pour former un noyau, sont un groupe alkylène en $C_1$ à $C_5$.

26. Composé selon la revendication 25, dans lequel $R^1$, $R^2$ et $R^5$ sont respectivement le groupe n-butyle.

27. Procéde pour la préparation d'un composé représenté par la formule (II)

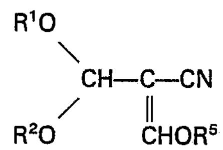

(II)

dans laquelle $R^1$, $R^2$ et $R^5$ peuvent être identiques ou différents et représentent chacun un groupe alkyle en $C_1$ à $C_5$, ou $R^1$ et $R^2$, liés l'un à l'autre pour former un noyau, sont un groupe alkylène en $C_1$ à $C_5$, qui comporte la réaction d'un composé représenté par la formule (V)

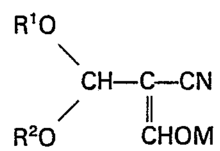

(V)

dans laquelle $R^1$ et $R^2$ ont les mêmes définitions que donnés ci-dessus, et M représente un métal alcalin, avec un alcool, de formule $R^5$—OH dans laquelle $R^5$ a la définition donnée ci-dessus et qui bout en formant un azéotrope avec l'eau, en présence d'un acide, tout en éliminant ledit alcool et l'eau produite du système de réaction par distillation azéotropique.

28. Procédé selon la revendication 27, dans lequel le métal alcalin est choisi parmi le sodium, le potassium, le lithium et le rubidium.

29. Procédé selon la revendication 27, dans lequel l'acide est choisi parmi l'acide sulfurique concentré, l'acide chlorhydrique concentré, l'acide chlorhydrique, l'acide phosphorique concentré, l'acide p-toluènesulfonique et l'acide acétique.

30. Procédé selon la revendication 27, dans lequel la quantité d'acide à utiliser est comprise dans l'intervalle allant de 1 à 10 équivalents par rapport au composé représenté par la formule (V).

31. Procédé selon la revendication 27, dans lequel l'alcool est choisi parmi l'éthanol, les propanols, les butanols et les pentanols.

32. Procédé selon la revendication 31, dans lequel l'alcool est le n-butanol.

33. Procédé selon la revendication 27, dans lequel la quantité de l'alcool à utiliser est comprise dans l'intervalle allant de 3 à 200 parties en poids pour une partie en poids du composé représenté par la formule (V).

34. Procédé selon la revendication 27, dans lequel on effectue la réaction pour former le composé représenté par la formule (II), sous pression réduite ou ambiante, à une température allant de 0 à 120°C.